(19) **Europäisches Patentamt**

**European Patent Office**

**Office européen des brevets**

(11) **EP 0 907 358 B1**

(12) **EUROPEAN PATENT SPECIFICATION**

(45) Date of publication and mention
of the grant of the patent:
**12.12.2001 Bulletin 2001/50**

(21) Application number: **97916120.5**

(22) Date of filing: **20.03.1997**

(51) Int Cl.[7]: **A61K 9/22**, A61K 9/52

(86) International application number:
**PCT/US97/04495**

(87) International publication number:
**WO 97/37640 (16.10.1997 Gazette 1997/44)**

(54) **UNIFORM DRUG DELIVERY THERAPY**

THERAPIE MITTELS GLEICHFÖRMIGER ARZNEISTOFFVERABREICHUNG

TRAITEMENT PAR ADMINISTRATION UNIFORME DE MEDICAMENTS

(84) Designated Contracting States:
**AT BE CH DE DK ES FI FR GB GR IE IT LI LU NL PT SE**

(30) Priority: **05.04.1996 US 14889 P**

(43) Date of publication of application:
**14.04.1999 Bulletin 1999/15**

(73) Proprietor: **ALZA CORPORATION**
**Palo Alto California 94303-0802 (US)**

(72) Inventors:
• **AYER, Atul, Devdatt**
**Palo Alto, CA 94303 (US)**
• **LAM, Andrew**
**San Francisco, CA 94122 (US)**
• **MAGRUDER, Judy, A.**
**Mountain View, CA 94040 (US)**
• **HAMEL, Lawrence, G.**
**Mountain View, CA 94040 (US)**
• **WONG, Patrick, S.-L.**
**Palo Alto, CA 94306 (US)**

(74) Representative: **Stebbing, Peter John Hunter et al**
**Ablett & Stebbing,**
**Caparo House,**
**101-103 Baker Street**
**London W1M 1FD (GB)**

(56) References cited:
**GB-A- 1 503 116**          **US-A- 4 327 725**
**US-A- 4 612 008**          **US-A- 4 765 989**
**US-A- 4 783 337**

**Description**

FIELD OF THE INVENTION

This invention pertains to a dosage form that provides a substantially uniform delivery of drug over an extended period of time. More particularly, the invention concerns a dosage form that provides a known and constant drug release pattern for an indicated therapy. The invention relates also to a dosage form that provides a controlled-constant and uniform delivery of a known dose of drug over time.

BACKGROUND OF THE INVENTION

A critical need exists for a dosage form for the controlled and uniform administration of a drug for therapy over time. Presently, in the practice of pharmacy and medicine, a drug is administered in conventional pharmaceutical forms, such as tablets and capsules. These conventional forms deliver their drug by dumping and this leads to uneven dosing of drug, to uneven blood levels of drug characterized by peaks and valleys, and accordingly this does not provide controlled and uniform therapy over time.

The prior art provided dosage forms for continuous therapy. For example, in United States Pat. No. 4,327,725 issued to Cortese and Theeuwes, and in United States Pat. Nos. 4,612,008; 4,765,989; and 4,783,337 issued to Wong, Barclay, Deters and Theeuwes, a dosage form is disclosed that provides therapy by generating an osmotic pressure inside the dosage form. The dosage form of these patents operate successfully for delivering a drug for a preselected therapy. With the delivery of some drugs however, these dosage forms often exhibit erratic release rate patterns, such as a nonuniform variation in the drug release rate, and the dosage form can stop delivering a drug, that is, the dosage form can shut-down intermittently.

It is immediately apparent, in view of the above presentation, that an urgent need exists for a reliable dosage form. The need exists for a dosage form endowed with properties for delivering a drug at a substantial and uniform rate over time. The need exists also for a dosage form substantially free-of-deviation in its release-rate profile, that delivers the needed dose of drug with a reduced amount of drug left in the dosage form at the end of the delivery period. It will be appreciated by those knowledgeable in the drug dispensing art, that is novel and unexpected dosage form is made available that provides a substantially uniform and known drug-release profile, free of the tribulations of the prior art, such a dosage form would represent an advancement and a valuable contribution in the drug dispensing art.

OBJECTS OF THE INVENTION

Accordingly, in view of the above presentation, it is an immediate object of the invention to provide a dosage form that delivers a drug in a substantially uniform dose to a biological drug receiving environment over an extended drug-delivery therapy time.

Another object of the invention is to provide a novel dosage form that substantially avoids administering a drug in a nonuniform and varying rate and therefore exhibits substantially the same dose-dispensing rate over time.

Another object of the invention is to provide a dosage form that delivers a predetermined and prescribed dose in the same manner over time while simultaneously lessen the amount retained or the residual drug left in and not delivered from the dosage form.

Another object of the invention is to provide a drug composition of matter comprising drug particles of 5 µm to 150 µm, micron, and hydrophilic polymer particles of 5 µm to 250 µm, characterized by the drug particles and the hydrophilic polymer particles functioning together to provide a uniform and nonvarying rate of release of both substantially-free of a deviation and substantially-free of a decrease in the rate of the release over time.

Another object of the invention is to provide a dosage form comprising a membrane that surrounds a drug core comprising drug particles of 1 to 150 µm and hydrophilic polymer particles of 1 to 250 µm, particles which are co-delivered from the dosage form through an exit formed by a process selected from the group consisting of a drilled exit, a bioerosion exit, a leaching exit, a solubilizing exit, and an exit formed by rupture.

Another object of the invention is to provide a dosage form comprising a membrane comprising a semipermeable composition that surrounds a core comprising a drug layer comprising drug particles of 1 to 150 µm and polymer particles of 1 to 250 µm, and a displacement layer comprising an osmopolymer-hydrogel that imbibes fluid, hydrates and increases in swelling volume and thereby displaces the drug layer through an exit membrane selected from an exit in the group consisting of an orifice, passageway, pore, microporous channel, porous overlay, porous insert, micropore, microporous membrane and porepassageway.

Another object of the invention is to make available a process for providing a substantially uniform and substantially nonvarying drug delivery program from a dosage form, wherein the process comprises the steps of selecting drug particles of 1 to 150 µm, selecting hydrophilic polymer particles of 1 to 250 µm, blending the selected particles into a drug-polymer core, and surrounding the core with a membrane comprising means for delivering the drug from the core in a substantially-uniform and substantially-nonvarying rate of release over a period of time up to 30 hours.

Another object of the invention is to provide a dosage form for delivering a drug to human, wherein the dosage form comprises a drug composition comprising 0.05 ng to 1.2g of drug having a particle size of 1 to 150

μm, and a hydrophilic polymer having a particle size of 1 to 250 μm, a push composition that imbibes fluid and expands for pushing the drug composition from the dosage form, a wall that surrounds the drug and the push composition and is permeable to the passage of fluid, an inner coat that surrounds the drug and push compositions positioned between the inside surface of the wall and the drug and push compositions for governing fluid imbition into the drug and push compositions for 30 minutes to 4 hours and 30 minutes, and at least one exit means in the wall for delivering the drug composition at a uniform and nonvarying rate over time.

Other objects, features, and advantages of the invention will be more apparent to those versed in the dispensing art comprising medicine and pharmacy from the following detailed specification taken in conjunction with the accompanying claims.

BRIEF DESCRIPTION OF THE FIGURES

Figure 1 illustrates the drug release rate variation with a drug possessing a particle size of 2 to 900 microns in the presence of a polymer possessing 25% and more of greater than 250 micron size.

Figure 2 illustrates the drug release rate variation from a dosage form with a drug size of less than 150 micron in the presence of a polymer possessing 25% and more of greater than 250 micron.

Figure 3 illustrates pronounced decrease in the variation of the drug release rate when the dosage form comprises a drug size of less than 150 micron accompanied by a polymer size of less than 250 micron.

DESCRIPTION OF THE INVENTION

The following examples are illustrative of the invention and they should not be considered as limiting the invention in any way, as these examples and other equivalents thereof will became apparent to those versed in the dispensing art in the light of the present specification and the accompanying claims.

EXAMPLE 1

A dosage form for delivering a drug orally to the gastrointestinal tract of the drug receiving patient in need of the drug's therapy is prepared as follows: first 5 mg of 135 μm amlodipine besylate, a calcium channel blocker, is blended with a 5% solution of poly (vinylpyrrolidone) of 30,000 number average molecular weight available from General Aniline and Film Corporation, New York, New York, in a fluid bed processor. Then, the granulated product is combined with 7.5 mg of 235 μm a poly (alkylene oxide), a poly(ethylene oxide), of 175,000 number average molecular weight available from the Union Carbide Corporation, Danbury, Connecticut, 0.5 mg of sodium chloride and 0.02 mg a stearic acid, and blended to provide a homogenous blend, by blending 35 rpm for 7 minutes. The homogenous blend is compressed into a drug composition and surrounded with a wall comprising a semipermeable composition and an exit forming agent.

The wall composition comprises 65 wt% cellulose acetate having an acetyl content of 34% and a 30,000 number average molecular weight dissolved in acetone:water, to which 1.8 wt% triacetin and 1.5 wt% sodium chloride are added with stirring constantly. The drug composition is sprayed in a fluidized bed air suspension coater to provide 10% wt wall. The dosage form is dried at 25°C for 18 hours. The dosage form releases the amlodipine besylate in a nonvarying rate through microchannels formed by fluid leaching of the sodium chloride in the gastrointestinal fluid of the patient.

EXAMPLE 2

The procedure of the above example is followed in this example, wherein in the present example the drug is selected from the group consisting of 5 mg of lisinopril indicated as an angiotensin converting enzyme inhibitor, 10 mg of buspirone hydrochloride indicated as an antianxiety drug, and 5 mg of oxybutynin hydrochloride indicated for relief of bladder instability, and wherein the lubricant is magnesium stearate and the semipermeable wall comprises mannitol.

EXAMPLE 3

A dosage form for the osmotically and hydrokinetically controlled release of a beneficial drug is made as follows: first, to a mixing bowl is added 500 mg of the oral antibacterial ciprofloxacin hydrochloride of 125 microparticle size followed by the addition of 105 mg of sodium carboxymethylcellulose of 22,000 number average molecular weight of 135 micron sizes and the ingredients mixed for 3 to 5 minutes to yield a homogenous mix. Next, 10 mg of 88 microcrystalline cellulose of 11,000 number average molecular weight is added to the mixing bowl and 0.05 mg of drug delivery surfactant sodium lauryl sulfate added to the bowl and all the ingredients mixed for 5 minutes. Then, an aqueous solution containing 7.5 mg of poly(vinylpyrrolidone) of 30,000 number average molecular weight is added with mixing and the resulting mixture is passed through an extruder onto a small tray and let dry overnight. The granulation is dried for 5 hours at 50°C and 0.03 mg of lubricant added with mixing for 1 minute. A solid fluid imbibing osmotic care is prepared in tablet press with a concave punch.

Next, an internal subcoat, drug free, is prepared comprising 94 wt% hydroxyethylcellulose of 90,000 number average molecular weight and 6 wt% polyethylene glycol in distilled water is coated around the drug composition and the subcoated drug composition is dried for 1 hour at 45°C. Then, an outer coat comprising a semipermeable composition and a pore-passageway former is prepared by adding cellulose acetate of 39.43% acetyl content to a cosolvent of methylene chloride and methanol to yield a solution effected by stirring and warming. Next, the pore-former sorbitol is added to a cosolvent of water and methanol with mixing followed by adding polyethylene glycol to produce the outer coating solution. Finally, the outer coating solution is coated around the subcoat in a pan coater and then dried for 18 hours at 45°C in a forced air oven, to yield the desired dosage form. The dosage form, in operation in the gastrointestinal fluid of a human in need of drug therapy, provides a uniform and nonvarying-order of drug release through exit passageways of controlled porosity effected by the fluidic leaching of the soluble pore-forming additive incorporated in the semipermeable outer coat. The cooperation of the drug particles and the hydrophilic polymer particles provides a viscous gel that pushes the drug through the exits at the given rate.

EXAMPLE 4

The procedure of the above example is followed, with the proviso in this example the therapeutic member is selected from the group consisting of 40 mg of simvastatin for lowering cholesterol, 75 mg of venlafaxine antidepressant, 20 mg of fluoxetine antidepressant, 20 mg of antianginal nifedipine, 40 mg of lovastatin indicated for lowering cholesterol, 20 mg of enalopril maleate an angiotensin converting enzyme inhibitor, 120 mg of diltiazem for managing calcium ion influx, 500 mg of ciprofloxacin hydrochloride an antibacterial, 100 mg of sertraline hydrochloride an oral antidepressant, 100 mg of cyclosporin an immunosuppressant, 1 mg of terazosin hydrochloride an alpha-adrenoceptor blocker, 50 mg of sumatriptan succinate a 5-hydroxytryptamine receptor agonist, 40 mg of pravastatin sodium a hypolipidemic, 500 mg of an anti-HIV-proteinase inhibitor such as nelfinavir, saquinavir, indinavir, or ritonavir, an anti-HIV such as zidovudine, didanosine, or lamivudine, a reverse transcriptase inhibitor such as loviride, an antiviral herpes such as fumciclovir or gancidovir, 10 mg of alendronate sodium for treating osteoporosis, and 2.5 mg of conjugated estrogen indicated for the treatment of vasomotor symptoms associated with menopause, atrophic vaginitis and osteoporosis loss of bone mass.

EXAMPLE 5

A dosage form for the oral uniform and nonvarying release of a drug to a biological drug receptor is manufactured as follows: first, 6000g of verapamil hydrochloride, indicated for the treatment of angina and high blood pressure, having nonuniform particle size distribution between 1 micron to 900 micron, 3047g of poly(ethylene oxide) having a number average molecular weight of 300,000 and having 25% particles greater than 250 micron, 500g of sodium chloride and 100g of poly(vinylpyrrolidone) having a number average molecular weight of 40,000 are added to a Freund Flo-Coater's bowl, a fluid bed granulator. The bowl is attached to the Flo-Coater and the granulation process is initiated. Next, the dry powders are air suspended and mixed for five minutes. Then, a solution prepared by dissolving 300g of poly(vinylpyrrolidone) having a number average molecular weight of 40,000 in 4,500g of water is sprayed from 2 nozzles onto the powder. The coating conditions are monitored during the poly(vinylpyrrolidone) solution spraying as follows: a total spray rate of 240 g/min from each nozzle, an inlet temperature of 45°C, an airflow of 1000 cfm. The coating process is computerized and automated in cycles. Each cycle contained 30 seconds of solution spraying followed by two seconds of drying and 10 seconds of filter bags with shaking to unglue any possible powder deposits. At the end of the solution spraying period, the coated granulated particles are continued in the drying process for 25 minutes. The machine is turned off, and the coated granules are removed from the coater. The coated granules are sized using a fluid air mill. The granulation is transferred to a mixer, mixed and lubricated with 50g of magnesium stearate and mixed with 4g of butylated hydroxytoluene, to provide the drug composition.

Next, a push-displacement composition is prepared as follows:
first, 7342g of poly(ethylene oxide) possessing a number average molecular weight of 7 million, 2000g of sodium chloride, 200g of hydroxypropylmethylcellulose of 11,200 number average molecular weight, 100g of black ferric oxide are added to the Freund Flo-Coater's bowl.
The bowl is attached to the Flo-Coater and the granulation process is started to effect the process. The dry powders are air suspended and mixed for six minutes. Then, a solution is prepared by dissolving 300g of hydroxypropylmethylcellulose having a number average molecular weight of 11,200 in 4,500g of water is sprayed from 2 nozzles onto the air suspended powder mix. The coating conditions were monitored during the hydroxypropylmethylcellulose spraying of the solution. The conditions are identical to those described in the above drug granulation process, except for the drying cycle of less than 25 minutes. The granulated powders are removed from the granulator and sized in a fluid air mill. The granulation is transferred to a blender, mixed and lubricated with 50g of magnesium stearate and with 8 grams of butylated hydroxytoluene to yield the push-displacement composition.

Next, the drug composition and the push composition are compressed into a bilayered core. First, 300 mg of the drug composition comprising 180 mg of verapamil hydrochloride is added to the punch and tamped, then 100 mg of the push displacement composition is added to the punch and the layers pressed under a pressure of 2200 pounds into a 13/32 inch (1.032 cm) diameter contacting, bilayered arrangement.

Next, the bilayered core is coated with a subcoat. The subcoat comprises 95% hydroxyethylcellulose of 90,000 number average molecular weight and 5% polyethylene glycol of 3350 average molecular weight. The ingredients are dissolved in water to make a 5% solid solution. The subcoat forming composition is sprayed onto and around the bilayer core in a 24 inch Vector Hi-Coater. The dry subcoat weighed 79 mg.

Next, the hydroxyalkylcellulose, a hydroxyethylcellulose, a subcoated bilayered cores are over coated with a semipermeable composition. The overcoat membrane forming composition comprises 60% cellulose acetate having an acetyl content of 39.8%, 35% hydroxypropylcellulose of 40,000 number average molecular weight and 5% polyethylene glycol of 3350 avg. molecular weight is dissolved in methylene chloride:methanol (90:10 wt:wt) cosolvent to make a 4% solid solution. The semipermeable membrane forming composition is sprayed onto and around the subcoated bilayer core. The semipermeable membrane, after drying weighed 43 mg.

Next, two 27 mil (0.686 mm) exit passageways are drilled through the outer semipermeable membrane and the inner subcoat to connect the drug layer with the exterior of the dosage form. The residual solvents are removed by drying for 96 hours at 50°C and 50% humidity. Finally, the dosage forms are dried for 2 hours at 50°C to remove any excess moisture.

The dosage form manufactured by this procedure comprises a drug composition with a weight of 300 mg, consisting of 180 mg of verapamil hydrochloride, 91.41 mg of poly (ethylene oxide) of 300,000 molecular weight, 12 mg of poly(vinylpyrrolidone) of 40,000 molecular weight, 15 mg of sodium chloride, 0.12 mg of butylated hydroxy toluene and 1.5 mg of magnesium stearate. A push-displacement composition that weighs 100 mg consisting of 73.5 mg of poly(ethylene oxide) of 7,000,000 molecular weight 20 mg of sodium chloride, 5 mg of hydroxypropylmethylcellulose of 11,200 molecular weight, 0.92 mg of black ferric oxide, 0.08 mg of butylated hydroxytolune and 0.5 mg of magnesium stearate. The dosage form subcoat weighed 78.8 mg consisting of 74.86 mg of hydroxyethylcellulose of 90,000 molecular weight and 3.94 mg of polyethylene glycol of 3350 molecular weight. The outer wall weighed 42.6 mg consisting of 25.56 mg of cellulose acetate of 39.8% acetyl content, 14.90 mg of hydroxypropylcellulose of 40,000 molecular weight, and 2.13 mg of polyethylene glycol of 3350 molecular weight. This dosage form had a $(dm/dt)_t$ mean release rate of 18.6 mg/hr between the fourth and ninth hour.

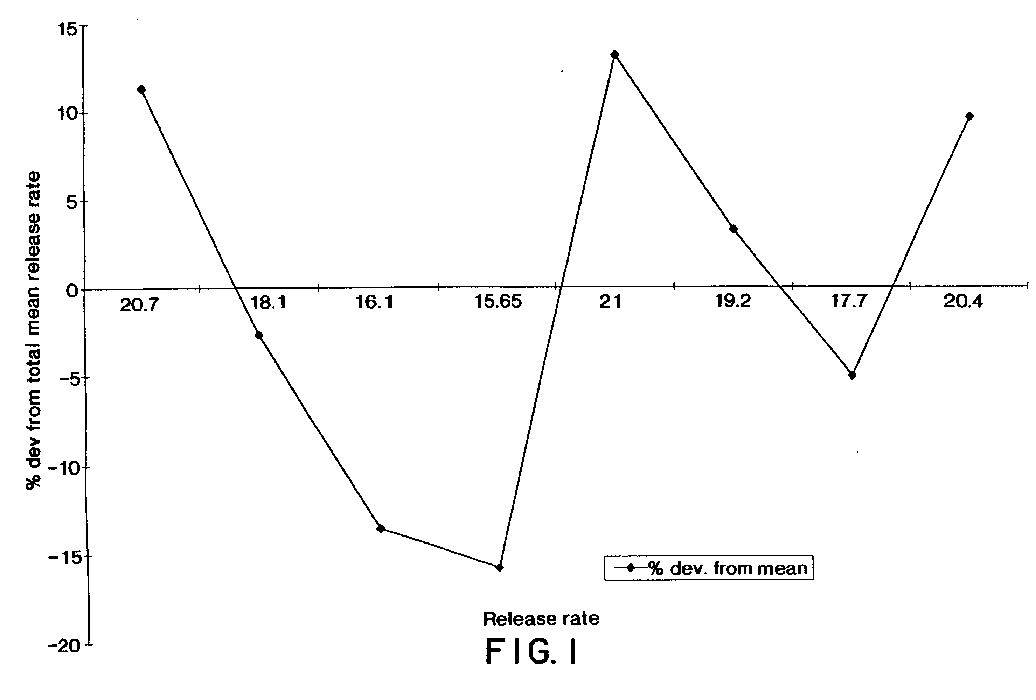

The delivery pattern for the dosage form prepared by this example is illustrated in figure 1. In figure 1, the nonuniform variability release rate is seen over the steady portion illustrated by the line starting at zero and extended to the right of the figure. The release rate variation is for a drug having a 1 to 900 micron particle size released in the presence of a hydrophilic polymer having greater than 25% particles larger then 250 micron. The solid line depicts the % deviation from the total mean release rate.

The mean release rate for a given dosage form is expressed by the number along the line starting at zero. In the figure No. 1 the erratic behavior is seen because the dosage form lacks uniform particles of a limited range.

The erratic behavior is characterized by a substantial deviation of individual system from the mean (dosage form) steady state release rate performance. This erratic behavior phenomena is attributed to the inability of the hydrophilic polymer, the poly(ethylene oxide), to carry and suspend large drug particles,(the verapamil hydrochloride), the difference in the hydration time between the large and small drug particles, and the larger hydrophilic polymer particles greater than 250 micron, which significantly changes the hydration and the drug suspending properties of the drug compositional layer that resulted into a large percent negative deviation in the $(dm/dt)_i$ from the $(dm/dt)_t$. The expression $(dm/dt)_t$ denotes the total mean release rate for all dosage forms in the zero portion, $(dm/dt)_i$ denotes the mean release rate of an individual dosage form in 4 to 9 hours, and (% dev)$_i$ denotes the percent deviation in an individual dosage form mean release rate, (4 to 9 hours) from the total mean release rate. The figure reports results obtained from the following equation:

$$(\% \ dev) = \frac{(dm/dt)_i - (dm/dt)_t}{(dm/dt)_t}$$

EXAMPLE 6

A dosage form for the delivery of a drug orally to a human is prepared as follows: first 6000g of verapamil hydrochloride having a particle size of less than 150 micron, 3047g of poly(ethylene oxide) possessing a number average molecular weight of 300,000 with 25% particles larger than 250 micron, 500g of sodium chloride, 100g of poly(vinylpyrrolidone) having a number average molecular weight of 40,000 are added to the bowl of a fluid bed granulator. The granulation is carried out for 7 to 10 minutes. Next, the dry powders are air suspended and mixed for five minutes. Then, a solution is prepared by dissolving 300g of poly(vinylpyrrolidone) of 40,000 number average weight in 4,500g of distilled water is sprayed from 2 nozzles onto the dry powder. The coating conditions are monitored during spraying as follows:

a total spray rate of 240 g/min from each nozzle, an inlet temperature of 45°C and a process airflow of 1000 cfm. The coated process is automated in cycles. Each cycle consist of 30 seconds of solution spraying followed by two seconds of drying and 10 seconds of filter bags shaking to unglue and possible powder deposits. At the end of the solution spraying time, the coated granulated particles are continued with the drying process for 25 minutes. The machine is turned off, and the coated granules were removed from the coater. The coated granules are sized using a fluid air mill, the granulation is transferred to a mixer, mixed and lubricated with 50 grams of magnesium stearate and mixed with 4g of butylated hydroxytoluene to provide the drug composition used for forming a layer in the bilayer core.

Next, a push composition is prepared as follows: first, 7342g of poly(ethylene oxide) of 7,000,000 number average molecular weight, 2000g of sodium chloride, 200g of hydroxypropylmethylcellulose of 11,200 number average molecular weight, and 100 grams of black ferric oxide are added to the bowl of a fluid bed granulator. The granulation process is started, and the dry powders are air suspended and mixed for 6 minutes. Then, a solution is prepared by dissolving 300g of hydroxypropylmethylcellulose possessing a 11,200 number average molecular weight in 4,500g of water that is sprayed onto the air suspended powder mix. The coating conditions are monitored during the spraying and the physical conditions are identical as described for the above drug granulation, except that the drying cycle was less than 25 minutes. The granulated powders are removed from the granulator.

The granules are sized in a fluid air mill, then transferred to a blender and lubricated while mixing with 50g of magnesium stearate and 8g of butylated hydroxytoluene to yield the push composition.

Next, the drug composition and the push composition are pressed into a bilayered core, with the layers in contacting arrangement. First, 400 mg of the drug composition comprising 240 mg of verapamil hydrochloride is added to a tablet punch and tamped, then 135 mg of the push composition is added to the punch and the layers are pressed under a pressure head of 2300 pound in a 7/16 inch (1.11 cm) diameter contacting, bilayered arrangement. The bilayered-core tablets are coated with a subcoat. The subcoat comprises 95% hydroxyalkylcellulose, a (hydroxyethylcellulose) of 90,000 molecular weight and 5% polyethylene glycol of 3350 molecular weight, dissolved in water to provide a 5% solid solution. The subcoat forming composition is sprayed onto the around the bilayered core in a coater. The dry subcoat weighed 93 mg.

Next, an outer coat is applied to the dosage form. The subcoated bilayered-core tablets are coated with a semipermeable-membrane wall.

The membrane forming composition comprises 60% cellulose acetate having a 39.8% acetyl content, 35% hydroxypropylcellulose of 40,000 molecular weight and 5% polyethylene glycol of 3350 molecular weight. The wall forming composition is dissolved in methylene chloride:methanol (90:10 wt:wt) cosolvent to make a 4% solid solution. The semipermeable-membrane wall forming composition is sprayed onto and around the subcoated bilayer core in a coater to provide a two-coated dosage form. The semipermeable membrane dry weighed 51 mg.

Next, two 27 mil (0.686 mm) exit passageways are drilled through the outer and inner coats to connect the drug layer with the exterior of the dosage form. The residual solvents are removed by drying for 96 hours at 50°C and 50% humidity. Then, the osmotic dosage forms are dried for 2 hours at 50°C to remove excess moisture.

The dosage form manufactured by this procedure comprises a drug composition with a weight of 400 mg, consisting of 240 mg of verapamil hydrochloride, 121.88 mg of polyethylene oxide of 300,000 molecular weight, 16 mg of poly(vinylpyrrolidone) of 40,000 molecular weight, 20 mg of sodium chloride, 2 mg of magnesium stearate and 0.16 mg of butylated hydroxytoluene. The push composition of the dosage form weighed 135 mg and consists of 99.23 mg of poly(alkylene oxide), poly(ethylene oxide) of 7,000,000 molecular weight, 27 mg of sodium chloride, 6.75 mg of hydroxypropylmethylcellulose of 11,200 molecular weight, 1.24 mg of ferric oxide, 0.675 mg magnesium stearate, and 0.108 mg of butylated hydroxytoluene. The inner subcoat weighed 93.1 mg and consists of 88.45 mg of the hydroalkylcellulose, hydroxyethylcellulose of 90,000 molecular weight and 46.55 mg of polyethylene glycol of 3350 molecular weight. The outer coat weighed 51.1 mg and consists of 30.66 mg of cellulose acetate of 39.8% acetyl content, 17.89 mg of hydroxypropylcellulose of 40,000 molecular weight and 2.57 mg of polyethylene glycol of 3350 molecular weight. The dosage form prepared by this example had a $(dm/dt)_t$ mean release rate of 27 mg/hr during hours 4 to 9.

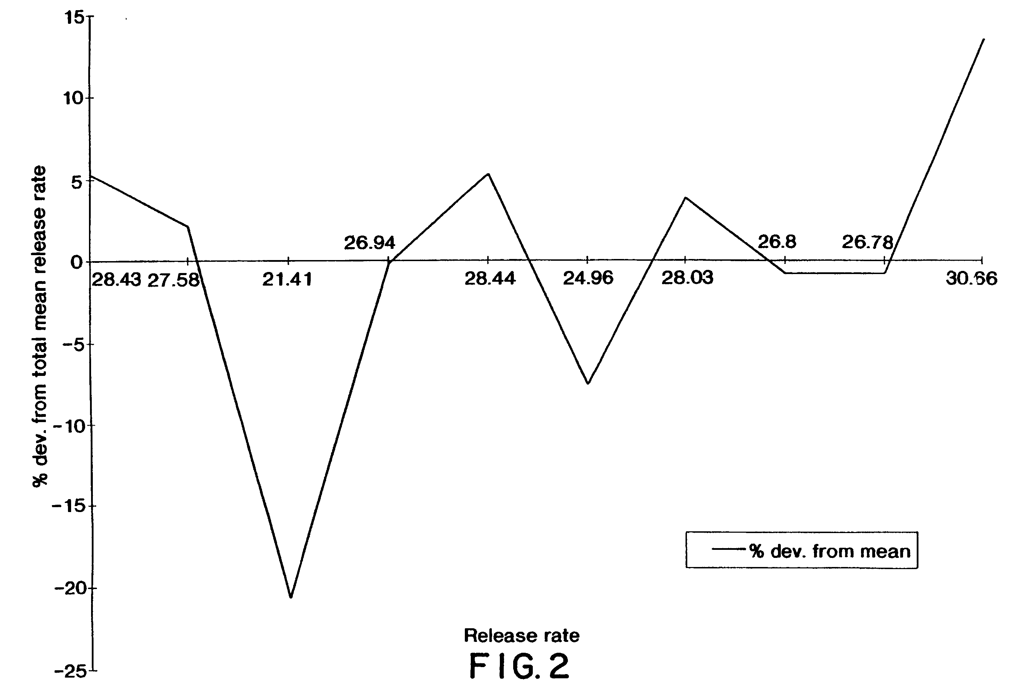

The drug delivery pattern for the dosage form prepared by this invention is seen in drawing figure 2. In figure 2, the nonuniform variability is depicted for the dosage form. The erratic release behavior is characterized by a substantial and pronounced deviation of individual dosage forms from the mean dosage form steady state rate performance. The figure denotes that larger polymer particles of from 250 micron significantly change the hydration and the drug carrying ability and suspension properties of the drug composition. This results in a large percent negative deviation in the expression $(dm/dt)_i$ from the expression $(dm/dt)_t$.

<u>EXAMPLE 7</u>

A dosage form for the delivery of a drug orally to the gastrointestinal tract of a human in need of drug therapy is prepared as follows: first, 6000g of verapamil hydrochloride having a particle size of 150 or smaller microns, 3047g of poly(ethylene oxide) of 300,000 molecular weight and having a particle of 250 or smaller microns, 500g of powdered sodium chloride, 100g of poly(vinylpyrrolidone) having a 40,000 molecular weight are added to a coater and granulated in air for five minutes. Next, a solution is prepared by dissolving 300g of poly(vinylpyrrolidone) of 40,000 molecular weight in 4,500g of water and sprayed onto the powder. The spray rate is 240g/min at an inlet temperature of 45°C and an airflow of 1000 cfm. The spraying is effected in two cycles consisting of 30 seconds of solution spraying followed by two seconds of drying and 10 seconds of shaking to unglue powder deposits.

At the end of the solution spraying period, the coated granulated particles are dried for an additional 25 minutes. Then, the coated granules are sized in a fluid air mill. The granulation is transferred to a mixer, and lubricated with 50g of magnesium stearate and with 4g of butylated hydroxytoluene, to yield the drug composition.

Next, a push displacement composition is prepared as follows:

first, 7342g of poly(ethylene oxide) of 7,000,000 molecular weight, 2000g of sodium chloride, and 2000g of hydroxypropylmethylcellulose of 11,200 molecular weight, and 100g (grams) of black ferric oxide are added to the bowl of a fluid bed granulator. The granulation is started and the powders mixed for six minutes. Then, a solution is prepared by dissolving 300g of hydroxypropylmethylcellulose of 11,200 molecular weight in water and sprayed onto the air suspended particles. The coating process is as described above. The granules are sized in a fluid air mill and transferred to a blender, and blended with 50g of magnesium stearate and 8g of butylated hydroxytoluene, to yield the push-displacement composition.

Next, the drug composition and the push composition are compressed into a bilayered tablet as follows: first, 400 mg of the drug composition containing 240 mg of verapamil hydrochloride is added and tamped, then it is overlayed with 135 mg of the push composition, and the two compositions pressed under 2300 pounds into a 7/16 inch (1.11 cm) diameter contacting, bilayered arrangement.

Next, the compressed bilayer tablets are coated with a subcoat laminate. The subcoat comprises 95% hydroxyethylcellulose of 90,000 molecular weight and 5% polyethylene glycol of 3350 molecular weight dissolved in distilled water to make a solid solution. The subcoat forming composition is sprayed onto and around the bilayered tablet in a coater to provide an encompassing laminate. The dry subcoat weighed 93 mg.

Next, the subcoat is overcoated with a semipermeable wall. The semipermeable composition comprises 60% cellulose acetate having an acetyl content of 39.8%, 35% hydroxypropylcellulose of 40,000 molecular weight and 5% polyethylene glycol of 3350 average molecular weight. The wall-forming composition is dissolved in a methylene-chloride:methanol (90:10 wt:wt) cosolvent to make a 4% solid solution. The semipermeable overcoat is sprayed onto and around to encase the subcoat. The semipermeable wall weighed 51 mg.

Next, two 27 mil (0.686 mm) exit passageway are drilled through the dual oats to connect the drug layer with the exterior of the dosage form.

The residual solvents are removed by drying for 96 hours at 50°C and 50% humidity. Next, the osmotic, fluid imbibing dosage forms are dried for 2 hours at 50°C to remove excess moisture.

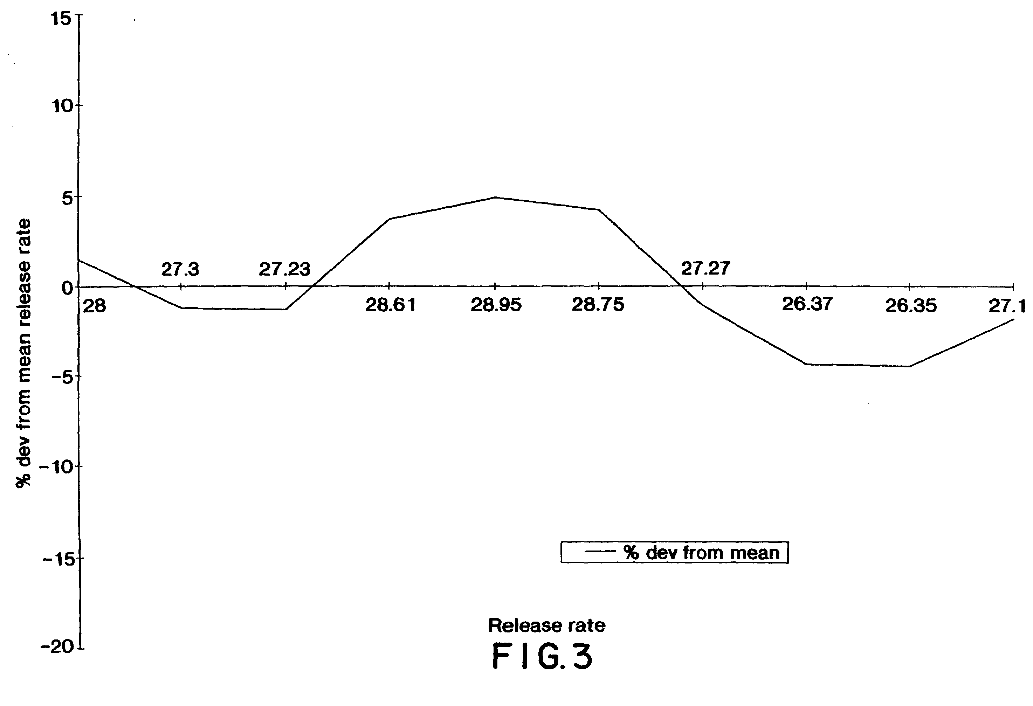

The dosage form prepared by this example embraces the same composition as the example immediately above, except for the controlled drug particle size and the controlled hydrophilic polymer particle size in the drug composition. This double particle control produces substantially uniform dose dispensing, substantially-free of a wide variation in the dose dispensing pattern. Accompanying figure 3 depicts the drug delivery pattern for this example. The figure depicts a release rate of $(dm/dt)_t$ equal to 27.9 mg/hr during hours 4 to 9. The figure illustrates that a nonuniform variability is not observed for the dosage form provided by this example.

## EXAMPLE 8

A dosage form prepared according to Example 8 wherein the drug in the dosage form is a calcium channel blocking drug selected from the group consisting of isradipine, nilvadipine, flunarizine, nimodipine, diltiazem, nicardipine, nitredipine, nisoldipine, filodipine, amlodipine, cinnarizine, and fendiline.

## EXAMPLE 9

The procedure described in the above is repeated in this example, with the processing conditions as previously set forth, except that, in this example the drug is an angiotensin converting enzyme inhibitor selected from the group consisting of alacipril, benazepril, cialzepril, captropril, delapril, enalapril, fosinopril, lisinopril, moveltypril, perindopril, quinapril, ramipril, spirapril, and zofenopril.

## EXAMPLE 10

The procedures of the above examples are followed in this example with the addition of the drug and is protected against oxidative attack and oxidation by adding to the processing drug composition 0.05 ng to 7 mg of an antioxidant selected from the group consisting of d-alpha tocopherol, dl-alpha tocopherol, d-alpha tocopherol acetate, d-alpha tocopherol acid succinate, dl-alpha tocopherol acid succinate, dl-alpha tocopherol palmitate, ascorbic acid, ascorbyl oleate, ascorbyl palmitate, butylated hydroxyanisole, butylated hydroxytoluene, sodium ascorbate, calcium ascorbate, and propyl gallate stabilizers.

## EXAMPLE 11

The procedures of the above examples are followed in this example with an addition to the drug composition comprising 0.05 ng to 7 mg of an antioxidant stabilizer and 0.05 ng to 7.5 mg of a lubricant selected from the group consisting of magnesium stearate, calcium stearate, magnesium oleate, magnesium palmitate, corn starch, potato starch, bentonite, citrus pulp, and stearic acid; and, with all the ingredients in the drug composition when expressed in weight percent equal to 100 wt% weight percent.

EXAMPLE 12

The procedures of the above examples are followed in this example with an addition to the drug composition of means protection the drug against daylight and ultraviolet light; wherein, the addition comprising adding to the drug composition 0.01 mg to 10 mg of surface-active agent selected from anionic, cationic, amphoteric and nonionic surfactants including dialkyl sodium sulfosuccinate, polyoxyethylene glycerol, polyoxyethylene stearyl ether, propoxy-ethoxy copolymer, polyoxyethylene fatty alcohol ester, polyoxyethylene fatty acid ester, ethoxylated hydrogenated castor oil, and butoxylated hydrogenated castor oil; and adding to the drug composition 0.01 mg to 10 mg of riboflavin to stabilize the drug against light.

ADDITIONAL DISCLOSURE OF THE INVENTION

In the specification and in the accompanying claims, the term beneficial agent also includes drugs. The term drug includes any physiologically or pharmacologically active substance that produces a local or a systemic effect, in animals, including warm-blooded mammals, humans and primates; avians, household, sport, and farm animals; laboratory animals; fishes; reptiles; and zoo animals. The term "physiologically" as used herein, generically denotes the administration of a drug to produce generally normal drug levels and functions. The term "pharmacologically" denotes generally variations in response to the amount of drug administered to a host. The drug can be in various forms such as unchanged molecules, molecular complexes, pharmacologically acceptable salts such as hydrochloride, hydrobromide, sulfate, laurate, palmitate, phosphate, nitrite, nitrate, borate, acetate, maleate, tartiate, oleate, salicylate, and the like. For acidic drugs, salts of metals, amines, or organic cations, for example quarternary ammonium can be used. Derivatives of drugs, such as bases, ester and amide can be used. A drug that is water insoluble can be used in a form that is water soluble derivative thereof, or as a base derivative thereof, which in either instance or in its delivery by the osmotic system, is converted by enzymes, hydrolyzed by the body pH, or by other metabolic processes to the original therapeutically active form. The amount of drug in a dosage form, that is, in the drug composition is 25 ng to 750 mg. The dosage form comprising the drug can be administered, once, twice, or thrice daily.

The active drug that can be delivered includes inorganic and organic compounds without limitation, including drugs that act on the peripheral nerves, adrenergic receptors, cholinergic receptors, nervous system, skeletal muscles, cardiovascular system, smooth muscles, blood circulatory system, synoptic sites, neuroeffector junctional sites, endocrine system, hormone systems, immunological system, organ systems, reproductive system, skeletal system, autocoid systems, alimentary and execretory systems, inhibitory of autocoids and histamine systems, and physiological systems. The active drug that can be delivered for acting on these animal systems includes depressants, beta-blockers, hypnotics, sedatives, psychic energizers, tranquilizers, anti-convulsants, muscle relaxants, steroids, antiparkinson agents, analgesics, anti-inflammatories, polypeptides, local anesthetics, muscle contractants, anti-microbials, antimalarials, hormonal agents, contraceptives, sympathomimetics, diuretics, anti-parasitics, neoplastics, hypoglycemics, ophthalmics, electrolytes, diagnostic agents, cardiovascular drugs, calcium channel blockers, angio-tensin-converting enzyme inhibitors, and the like.

Exemplary of drugs that can be delivered from the dosage form of this invention include a drug selected from the group consisting of amifostine, prochlorperazine edisylate, ferrous sulfate, aminocaprioc acid, potassium chloride, mecamylamine hydrochloride, procainamide hydrochloride, amphetamine sulfate, benzphetamine hydrochloride, isoproternal sulfate, methamphetamine hydrochloride, phenmetrazine hydrochloride, bethanechol chloride, methacholine chloride, pilocarpine hydrochloride, antropine sulfate, methascopolamine bromide, isopropamide iodide, tridihexethyl chloride, phenformin hydrochloride, methylphenidate hydrochloride, oxprenolol hydrochloride, metroprolol tartrate, cimetidine hydrochloride, diphenidol, meclizine hydrochloride, prochlorperazine maleate, phenoxybenzamine, thiethylperzine, maleate, anisindone, diphenadione erythrityl teranitrate, dizozin, isofurophate, reserpine, acetazolamide, methazolamide, bendroflumenthiazide, chlorpropamide, tolazamide, chlormadinone acetate, phenaglycodol, allopurinol, aluminum aspirin, methotrexate, acetyl sulfisoxazle, erythromycin, progestins, estrogenic progrestational, corticosteroids, hydrocortisone acetate, cortisone acetate, triamcinolone, methyltesterone, 17β-estradiol, ethinyl estradiol, ethinyl estradiol 3-methyl ether, prednisolone, 17-hydroxyprogesterone acetate, 19-nor-progesterone, norgestrel, norethindone, norethiderone, progesterone, norgestrone, orethynodrei, aspirin, indomethacin, aproxen, fenoprofen, sulidac, diclofenac, indoprofen, nitroglycerin, propranolol, metroprolol, vallproate, oxyprenolol, timolol, atenolol, alpreholol, cimetidine, clonidine, imipramine, levodopa, chloropropmazine, resperine, methyldopa, dihydroxyphenyllalanine, pivaloyloxyethyl ester of ε-methyldopa hydrochloride, theophylline, calcium gluconate ferrous lactate, ketoprofen, ibuprofen, cephalexin, erythromycin, haloperiodol, zomepirac, vincamine, diazepam, phenoxybenzamine, β-blocking agents; calcium-channel blocking drugs such as nifedipine, diltiazem, isradipine, nilvadipine verapamil, flunarizine, nimodipine, felodipine, amlodipine, cinnarizine and fendiline; angiotensin converting enzyme inhibitors selected from the group consisting of angiotensin converting enzyme inhibitors that are essentially free of sulfur, angiotensin converting enzyme inhibitors containing a sulfhydryl group, angiotensin converting enzyme inhibitors containing a linear sulfide, angiotensin converting enzyme inhibitors containing a cyclic sulfide angiotensin converting enzyme inhibitors containing a methylsulfonyl group and angiotensin enzyme inhibitors represented by a member selected from the group consisting of ramipril, fosinopril, altiopril, benazepril, libenzapril, alacepril, citazapril, cilazaprilate, perindopril, zofenopril, enalapril, lisinopril, imidapril, spirapril, rentrapril, captopril, delapril, alindapril, indolapril, and quin-april; propranolol, naproxen, phenylpropanolamine, gli-pizide, venlafaxine, and beneficial drugs known to the dispensing arts in Pharmaceutical Sciences, 1990, edited by Remington 18th Edition published by Mack Publishing Co., Easton, PA; Physicians' Desk Reference, 50th Edition, (1996) published by Medical Economics Co., Montvale, NJ, and, USP Dictionary, 1995, published by the United States Pharmacopeial Convention, Inc., Rockville, Maryland.

The dosage form of the invention is provided with at least one exit means. The exit means cooperates with the drug core for the uniform and substantially nonvarying drug-dose release from the dosage form. The exit means can be provided during manufacture of the dosage form, or the exit means can be provided during drug delivery by the dosage form in fluid environment of use. The expression exit means, as used for the purpose of this invention, included a member selected from the group consisting of passageway, aperture, orifice, bore, pore, micropore, porous element through which a drug can be pumped, diffuse, travel, or migrate, a hollow fiber, capillary tube, porous insert, porous overlay, microporous member, and porous composition. The expression includes also a compound or polymer that erodes, dissolves or is leached from the outer coat or wall, or from the inner coat to form at least one exit, or a multiplicity of exits.

The compound or polymer includes an erodible poly (glycolic) acid or poly (lactic) acid in the outer or inner coats, a gelatinous filament, a water-removable poly (vinyl alcohol), a leachable compound such as a fluid removable pore-former selected from the group consisting of an inorganic, organic, acid, salt, oxide, and carbohydrate. An exit or a plurality of exits can be formed by leaching a member selected from the group consisting of sorbitol, lactose, fructose, glucose, mannose, galactose, talose, sodium chloride, potassium chloride, sodium citrate, and mannitol; to provide an uniform-release dimensioned pore-exit means. The exit means can have any shape such as round, triangular, square, elliptical and the like for the uniform-metered dose release of a drug from the dosage form. The dosage form can be constructed with one or more than one exits in spaced apart relation or one or more than one surface of the dosage form. The exit means can be performed by drilling including mechanical and laser drilling through the outer, or inner or through both coats. Exits and equipment for forming exits are disclosed in U.S. Pat. Nos. 3,845,770 and 3,916,899 by Theeuwes and Higuchi; in U.S. Pat. Nos. 4,063,064 by Saunders, et al; and in U. S. Pat. No. 4,088,864 by Theeuwes, et al. Exit means comprising dimension, sized, shaped and adapted as a releasing-pore formed by aqueous leaching to provide a drug releasing pore are disclosed in U.S. Pat. Nos. 4,200,098 and 4,285,987 by Ayer and Theeuwes.

The particles used for the purpose of this invention are produced by comminution that produces the size of the drug and the size of the accompanying hydrophilic polymer used according to the mode and the manner of the invention. The means for producing particles include spray drying, sieving, lyophilization, sieving, crushing, grinding, jet milling micronizing and chopping to produce the intended micron particle size.

The process can be performed by size reduction equipment such as micropulverizer mill, fluid energy grinding mill, grinding mill, roller mill, hammer mill, attrition mill, chaser mill, ball mill, vibrating ball mill, impact pulverizer mill, centrifugal pulverizer, coarse crusher and fine crusher.

The size of the particle can be ascertained by screening including grizzly screen, flat screen, vibrating screen, revolving screen, shaking screen, oscillating screen and reciprocating screen. The processes and the equipment for preparing particles are disclosed in Pharmaceutical Sciences by Remington, 17th Ed., pg. 1585-1594, (1985); Chemical Engineers; Handbook, by Perry, Sixth Edition, pg. 21-13 to 21-19 (1984); Journal of Pharmaceutical Sciences, by Parrot, Vol. 61, No., 6, pg. 813 to 829 (1974); and Chemical Engineer, by Hixon, pg. 94 to 103, (1990).

In accordance with the practice of this invention, it has now been found the dosage can be provided with a semipermeable wall, also identified for the purpose of this invention as an outercoat. The semipermeable wall is permeable to the passage of an external fluid such as water and biological fluids, an it is substantially impermeable to the passage of a beneficial agent, as osmogent, an osmopolymer, and the like. The selectively semipermeable compositions used for forming the wall are essentially non-erodible and they are insoluble in biological fluids during the life of the dosage form.

Representative polymers for forming the wall comprise semipermeable homopolymers, semipermeable copolymers, and the like. In one presently preferred embodiment, the compositions comprise cellulose esters, cellulose ethers, and cellulose ester-ethers. The cellulosic polymers have a degree of substitution, D.S. of their anhydroglucose unit from greater than 0 up to 3 inclusive. By degree of substitution is meant the average number of hydroxyl groups originally present on the anhydroglucose unit that are replaced by a substituting group, or converted into another group.

The anhydroglucose unit can be partially or completely substituted with groups such as acyl, alkanoyl, alkenoyl, aroyl, alkyl, alkoxy, halogen, carboalkyl, alkylcarbamate, alkylcarbonate, alkylsulfonate, alkysulfamate, semipermeable polymer forming groups, and the like.

The semipermeable compositions typically include a member selected from the group consisting of cellulose acylate, cellulose diacylate, cellulose triacylate, cellulose triacetate, cellulose acetate, cellulose diacetate, cellulose triacetate, mono-, di- and tri-cellulose alkanylates, mono-, di-, and tri-alkenylates, mono-, di-, and tri-aroylates, and the like. Exemplary polymers include cellulose acetate have a D.S. of 1.8 to 2.3 and an acetyl content of 32 to 39.9%; cellulose diacetate having a D.S. of 1 to 2 and an acetyl content of 21 to 35%, cellulose triacetete having a D.S. of 2 to 3 and an acetyl content of 34 to 44.8%, and the like. More specific cellulosic polymers include cellulose propionate having a D.S. of 1.8 and a propionyl content of 38.5%; cellulose acetate propionate having an acetyl content of 1.5 to 7% and an acetyl content of 39 to 42%; cellulose acetate propionate having an acetyl content of 2.5 to 3%, an average propionyl content of 39.2 to 45%, and a hydroxyl content of 2.8 to 5.4%; cellulose acetate butyrate having a D.S. of 1.8, an acetyl content of 13 to 15%, and a butyryl content of 34 to 39%; cellulose acetate butyrate having an acetyl content of 2 to 29%, a butyryl content of 17 to 53%, and a hydroxyl content of 0.5 to 4.7%; cellulose triacylates having a D.S. of 2.6 to 3 such as cellulose trivalerate, cellulose trilamate, cellulose tripalmitate, cellulose trioctanote, and cellulose tripropionate; cellulose diesters having a D.S. of 2.2 to 2.6 such as cellulose disuccinate, cellulose dipalmitate, cellulose dioctanoate, cellulose dicarpylate, and the like; mixed cellulose esters such as cellulose acetate valerate, cellulose acetate succinate, cellulose propionate succinate, cellulose acetate octanoate, cellulose valerate palmitate, cellulose acetate heptonate, and the like. Semipermeable polymers are known in US Pat. No. 4,077,407 and they can be synthesized by procedures described in Encyclopedia of Polymer Science and Technology, Vol. 3, pages 325 to 354, 1964, published by Interscience Publishers, Inc., New York.

Additional semipermeable polymers for forming the outer wall comprise cellulose acetaldehyde dimethyl acetate; cellulose acetate ethylcarbamate; cellulose acetate methyl carbamate; cellulose dimethylaminoacetate; semipermeable polyamide; semipermeable polyurethanes; semipermeable sulfonated polystyrenes; cross-linked selectively semipermeable polymers formed by the coprecipitation of a polyanion and a polylcation as disclosed in U.S. Pat. Nos. 3,173,876; 3,276,586; 3,541,005; 3,541,006; and 3,546,142; semipermeable polymers as disclosed by Loeb et al in U.S. Pat. No. 3,133,132; semipermeable polystyrene derivatives; semipermeable poly (sodium styrenesulfonate); semipermeable poly (vinylbenzyltremethylammonium chloride); semipermeable polymers, exhibiting a fluid permeability of $10^{-5}$ to $10^{-2}$ (cc. mil/cm hr.atm) expressed as per atmosphere of hydrostatic or osmotic pressure differences across a semipermeable wall. The polymers are known to the art in U.S. Pat. Nos. 3,845,770; 3,916,899; and 4,160,020; and in Handbook of Common Polymers, by Scott, J.R., and Roff, W.J., 1971, published by CRC Press, Cleveland, Ohio.

The subcoat of the invention is in contacting position with the inner surface of the semipermeable wall, which outer semipermeable wall surrounds and encases the inner subcoat. The inner subcoat is 0.01 mm to 3 mm thick and it comprises a member selected from group consisting of hydroxyalkyl, hydroxyethylcellulose, hydroxyisopropylcelluose, hydroxybutylcellulose, and hydroxyphenylcellulose. The hydroxyalkylcellulose comprises a 9,500 to 1,250,000 number average molecular weight.

The drug composition comprised a hydrophilic polymer for providing in the drug composition a hydrophilic polymer particle that contributes to the uniform and nonvarying drug delivery pattern. Representatives of these polymers comprise a member selected from the group consisting of a poly (alkylene oxide) of 100,000 to 750,000 number average molecular weight including poly (ethylene oxide), poly (methylene oxide), poly (butylene oxide), and poly (hexylene oxide); and a poly (carboxymethylcellulose) of 40,000 to 400,000 number average molecular weight represented by poly (alkali carboxymethylcellulose), poly (sodium carboxymethylcelluose), poly (potassium carboxymethylcellulose), and poly (lithium carboxymethylcellulose). The drug composition can comprise a hydroxypropylalkylcellulose of 9,200 to 125,000 number average molecular weight for enhancing the delivery properties of the dosage form as represented by hydroxypropylethylcellulose, hydroxypropylmethylcellulose, hydroxypropylbutylcellulose, and hydroxypropylpentylcellulose; and a poly (vinylpyrrolidone) of 7,000 to 75,000 number average molecular weight for enhancing the flow properties of the dosage form.

The push-displacement composition in contacting layered arrangement comprised a polymer that imbibes an aqueous or biological fluid and swells to push the drug composition through the exit means from the dosage form. Representative of fluid-imbibing displacement polymers comprise a member selected from the group consisting of a poly (alkylene oxide) of 1,000,000 to 15,000,000 number average molecular weight as represented by poly (ethylene oxide) and a poly (alkali carboxymethylcellulose) of 500,000 to 3,500,000 number average molecular weight wherein the alkali is sodium, potassium or lithium. Examples of further polymers for formulation, the push-displacement composition comprise osmopolymers comprise polymers that form hydrogels such as Carbopol® acidic carboxypolymer, a polymer of acrylic and cross-linked with a polyallyl sucrose, also known as carboxypolymethylene and carboxyvinyl polymer having a molecular weight of 250,000 to 4,000,000; Cyanamer® polyacrylamides; cross-linked water swellable indenemaleic anhydride polymers; Good-rite® polyacrylic acid having a molecular weight of 80,000 to 200,000; Aqua-Keeps® acrylate polymer polysaccharides composed of condensed glucose units such as diester cross-linked polygluran; and the like. Representative polymers that form hydrogels are known to the prior art in U.S. Pat. No. 3,865,108 issued to Hartop; U.S. Pat No. 4,002,173 issued to Manning; U.S. Pat. No. 4,207,893 issued to Michaels; and in Handbook of Common Polymers, by Scott and Roff, published by the Chemical Rubber Co., Cleveland, Ohio.

The osmagent, also known as osmotic solute and as osmotically effective agent, that exhibits an osmotic pressure gradient across the outer wall and subcoat comprises a member selected from the group consisting of sodium chloride, potassium chloride, lithium chloride, magnesium sulfate, magnesium chloride, potassium sulfate, sodium sulfate, lithium sulfate, potassium acid phosphate, mannitol, urea, inosital, magnesium succinate, tartaric acid raffinore, sucrose glucose, lactose, sorbitol, inorganic salts, organic salts and carbohydrates.

Exemplary solvents suitable for manufacturing the hydroactivated layer and the wall comprise inert inorganic solvents that do not adversely harm the materials, the capsule, and the final laminated wall hydro-activated layer. The solvents broadly include members selected from the group consisting of aqueous solvents, alcohols, ketones, esters, ethers, aliphatic hydrocarbons, halogenated solvents, cycloaliphatic, aromatics, heterocyclic solvents and mixtures thereof. Typical solvents include acetone, diacetone alcohol, methanol, ethanol, isopropyl alcohol, butyl alcohol, methyl acetate, ethyl acetate, isopropyl acetate, n-butyl acetate, methyl isobutyl ketone, methyl propyl ketone, n-hexane, n-heptane, ethylene glycol monoethyl ether, ethylene glycol monothyl acetate, methylene dichloride, ethylene dichloride, propylene dichloride, carbon tetrachloride nitroethane, nitropropane tetrachloroethane, ethyl ether, isopropyl ether, cyclohexane. cyclooctane, benzene, toluene, naphtha, 1,4-dioxane, tetrahydrofuran, diglyme, water, aqueous solvents containing inorganic salts such as sodium chloride, calcium chloride, and the like, and mixture thereof such as acetone and water, acetone and methanol, acetone and ethyl alcohol, methylene dichloride and methanol, and ethylene dichloride and methanol.

The semipermeable wall and the subcoat of the dosage form can be formed in one technique using the air suspension procedure. This procedure consists of suspending and tumbling the bilayer core in a current of air, an inner subcoat composition and an outer semipermeable wall forming composition, until in either operation the subcoat and the outer wall coat is applied to the bilayer core. The air suspension procedure is well-suited for independently forming the wall of the dosage form. The air suspension procedure is described in U. S. Pat. No. 2,799,241; in J. Am. Pharm. Assoc., Vol. 48, pp. 451 to 459, (1959); and, ibid, Vol. 49, pp. 82 to 84, (1960).

The dosage form also can be coated with a Wurster® air suspension coater, using for example, methylene dichloride methanol as a cosolvent.

An Aeromatic® air suspension coater can be used employing a cosolvent. Other coating techniques, such as pan coating, can be used for providing the dosage form. In the pan coating system, the subcoat on the wall forming compositions are deposited by successive spraying of the respective compensation on the bilayered core accompanied by tumbling in a rotating pan. A pan coater is used because of its availability at commercial scale. Other techniques can be used for coating the drug core. Finally, the wall or coated dosage form are dried in a forced air oven at 40°C. for a week, or in a temperature and humidity controlled oven for 24 hours at 40°C. and 50% relative humidity to free the dosage form of solvent.

The dosage form of the invention is manufactured by standard techniques. Fro example, in one manufacture, the beneficial drug and other ingredients comprising the first layer facing the exit means are blended and pressed into a solid layer. The layer possesses dimensions that correspond to the internal dimensions of the area the layer is to occupying the dosage form and it also possesses dimensions corresponding to the second layer for forming a contacting arrangement therewith. The drug and other ingredients can be blended also with a solvent and mixed into a solid or semisolid form by conventional methods, such as ballmilling, calendering, stirring or rollmilling, and then pressed into a preselected shape. Next, a layer of osmopolymer composition is placed in contact with the layer of drug in a like manner.

The layering of the drug formulation and the osmopolymer layer can be fabricated by conventional two-layer press techniques. The two contacted layers are first coated with a subcoat and an outer semipermeable wall. The air suspensions and air tumbling procedures comprises in suspending and tumbling the pressed, contacting first and second layers in a current of air containing the delayed-forming composition until the first and second layers are surrounded by the wall composition.

In another manufacture, the dosage form is manufactured by the wet granulation technique. In the wet granulation technique, the drug and the ingredients comprising the first layer or drug composition, are blended using an organic solvent, such as denature anhydrous ethanol, as the granulation fluid. The ingredients forming the first layer or drug composition are individually passed through a preselected screen and then thoroughly blended in a mixer. Next, other ingredients comprising the first layer can be dissolved in a portion of the granulation fluid, the solvent described above. Then, the latter prepared wet blend is slowly added to the drug blend with continual mixing in the blender. The granulating fluid is added until a wet blend is produced, which wet mass blend is then forced through a predetermined screen onto oven trays. The blend is dried for 18 to 24 hours at 24°C. to 35°C. in a forced air oven. The dried granules are then sized. Next, magnesium stearate is added to the drug granulation, it is then put into milling jars and mixed on a jar mill for 10 minutes. The composition is pressed into a layer, for example, in a Manesty® press. The speed of the press is set at 20 rpm and the maximum load set at 2 tons. The first layer is pressed against the composition forming the second layer and the bilayer tablets are fed to the Kilian® dry Coata press and surrounded with the drug-free coat, followed by the exterior wall solvent coating.

Another manufacturing process that can be used for providing the compartment-forming composition comprises blending the powdered ingredients in a fluid bed granulator. After the powdered ingredients are dry blended in the granulator, a granulating fluid, for example, poly(vinylpyrrolidone) in water, is sprayed onto the powders. The coated powders are then dried in the granulator. This process granulates all the ingredients present therein while adding the granulating fluid. After the granules are dried, a lubricant such as stearic acid or magnesium stearate is mixed into the granulation, using a V-blender. The granules are then pressed in the manner described above.

## METHOD OF PRACTICING THE INVENTION

The invention provides a process for the substantially uniform and substantially nonvarying rate of release of a drug from a dosage form, herein the dosage form comprises a composition, a dose of drug in the composition, and a hydrophilic polymer in the composition, and wherein the process comprises (1) formulating the composition with a drug possession, a particle size up to and including 150 microns, and (2) formulating the composition with a hydrophilic polymer possessing a particle size up to and including 250 microns, hereby, through the copresence of (1) and (2) in the composition, the drug is delivered as the substantially uniform and nonvarying rate of release from the dosage form.

The invention provides also a process for substantially uniform and substantially nonvarying rate of release of a drug from a dosage form, wherein the dosage form comprises a composition, a dose of drug in the composition, a hydrophilic polymer in the composition, and a composition for displacing the drug composition from the dosage form, and wherein the process comprises (1) formulating the composition with a drug possessing a particle size up to and including 150 micron, (2) formulating the composition with a hydrophilic polymer possessing a particle size up to and including 150 microns, whereby through the copresence of (1) and (2) in combination with the composition for displacing the drug composition imbibing fluid, expanding and displacing the drug composition from the dosage form. The drug is delivered at a substantially uniform and nonvarying rate of release over time.

The invention comprises also a method for delivering a drug to a patient, wherein the method comprises: (A) admitting orally into the patient a dosage form comprising: (1) a semipermeable wall that surrounds and forms a compartment; (2) a drug composition in the compartment; (3) a dose of drug particles up to 150 micron in the drug composition; (4) a hydrophilic polymer of up to 250 micron in the drug composition; (5) an exit in the semipermeable wall; (B) imbibing fluid through the semipermeable wall into the drug composition whereby through the coaction of (2) and (3) a dispensable drug composition is formed in the dosage form; and (C) delivering the drug composition through the exit to a patient at a substantially uniform and nonvarying dose over time.

The invention comprises further a method for providing a drug-free interval by placing a subcoat in the dosage form in contact with the inside surface of the semipermeable wall and surrounding the drug composition, or surrounding both a drug composition and a push composition, which drug-free interval is followed in 2 to 5 hours by a drug delivery period of 1 to 15 hours. The latter method is indicated for the treatment of hypertension and angina as it provides a drug-free interval when a patient is less active, thus, at rest or when asleep, and the inventive method then provides drug during the rising and waking hours mainly during the time when activity reaches a maximum during the daytime hours.

The method of the invention pertains also to the management of blood pressure, the management of the systemic physiology, and to the management of chronotherapy, that is timetherapy by administering a drug according to the mode and the manner of the invention.

The novel dosage form of this invention uses dual means for the attainment of precise release rate of drugs that are difficult to deliver in the environment of use, while simultaneously maintaining the integrity and the character of the system. While there has been described and pointed out features and advantages of the invention, as applied to the presently preferred embodiments, those skilled in the dispensing art will appreciate that various modifications, changes, additions, and omissions in the system illustrated and described can be made without departing from the spirit of the invention.

## Claims

1. A process for providing a substantially uniform drug rate of release from a dosage form, wherein the dosage form comprises a composition comprising a dose of drug and a hydrophilic polymer; wherein the process comprises (1) formulating the composition with a drug possessing a size less than 150 micron, and (2) formulating the composition with a hydrophilic polymer of less than 250 micron; whereby, through the copresence of (1) and (2) in the composition, the drug is delivered at a substantially uniform rate of release from the dosage form.

2. A process according to Claim 1, wherein the composition is enveloped by a wall comprising means for releasing the drug from the dosage form.

3. A process according to either Claim 1 or 2, wherein the composition is surrounded by an outer wall and an inner subcoat, with means in the dosage form for releasing the drug from the dosage form.

4. A process for providing a substantially uniform drug rate of release from a dosage form, wherein the dosage form comprises: a drug layer comprising a dose of drug and a hydrophilic polymer and a dispensing layer comprising means for dispensing the drug layer from the dosage form; wherein the process comprises formulating the drug layer with a drug possessing a particle size up to 150 microns and a hydrophilic polymer possessing a particle size up to 250 microns; which, through the cooperation of the drug particles and the hydrophilic polymer particles and the dispensing layer assisting the drug layer, the drug is delivered at a substantially uniform rate of release from the dosage form.

5. A process according to Claim 4, wherein the hydrophilic polymer particle cooperates with the drug particle as a pharmaceutical carrier for delivering the drug from the dosage form.

6. A process according to either Claim 4 or 5, wherein the dispensing layer assists in displacing the drug layer from the dosage form.

7. A process according to any of Claims 4 to 6, wherein a wall encases both the drug layer and the dispensing layer and comprises means for releasing the drug from the dosage form.

8. A process according to any of Claims 4 to 7, wherein a wall surrounds the drug layer and the dispensing layer, and a subcoat between the wall and the drug layer and the dispensing layer, and the dosage form comprises exit means for releasing the drug from the dosage form.

9. A dosage form for the delivery of a drug, wherein the dosage form comprises:

   (a) a composition;

   (b) a dose of drug of less than 150 microns in the composition;
   (c) a hydrophilic polymer of less than 250 microns in the composition;
   (d) a wall comprising a composition permeable to the passage of fluid that surrounds the dose of drugs and the hydrophobic polymer; and
   (e) means in the wall for delivering the drug at a substantially uniform rate from the dosage form.

10. A dosage form for the delivery of a drug, wherein the dosage form comprises

   (a) a drug composition;
   (b) a dose of drug of less than 150 microns in the drug composition;
   (c) a hydrophilic polymer of less than 250 microns in the drug composition;
   (d) a coat that surrounds the drug composition comprising means for delaying release of drug from the drug composition;
   (e) a wall comprising a composition that surrounds the coat; and,
   (f) means in the dosage form for delivering the drug from the dosage form over time.

11. A dosage form for delivering a drug orally to a patient in need of the drug, wherein the dosage form comprises:

   (a) a drug composition comprising a drug having a particle size up to and including 150 microns, and a hydrophilic polymer carrier having a particle size up to and including 250 microns for the drug;
   (b) a displacement composition in contact with the drug composition comprising a polymer that expands in the presence of fluid for displacing the drug composition from the dosage form;
   (c) a wall that surrounds the drug and displacement composition and is permeable to the passage of fluid; and,
   (d) means in the dosage form for delivering the drug from the dosage form at a substantially uniform rate over time.

12. A dosage form according to claim 11 wherein a coat free of drug is disposed between the wall, and the drug and the displacement composition, thereby surrounding the drug and the displacement composition in order to slow the passage of fluid into the dosage form.

13. A dosage from according to any of Claims 9 to 12, wherein the drug is selected from at least one of verapamil, nifedipine, nilvadipine, flunarizine, nimodipine, diltiazem, nicardipine, nitredipine, nisoldipine, felodipine, amlodipine, isradipine, cinnarizini, fendiline, ramipril, fusinopril, altiopril, benazepril, libenzapril, alacepril, cialzapril, cilazaprilat, perindopril, zofenopril, inalapril, lisinopril, imidapril, spirapril, rentiapril, captopril, delapril, olindapril, indalapril and quinapril.

14. A dosage form according to any of Claims 9 to 12 wherein the drug is selected from at least one of prazosin, clonidine, pinacidil, alfuzosin and enalaprilat.

15. A dosage form according to any of Claims 9 to 12 wherein the drug composition further comprises an antioxidant.

16. A dosage form according to any of Claims 9 to 12, wherein the drug composition further comprises a surfactant.

17. A dosage form according to Claim 11, wherein the drug is selected from at least one of a calcium channel blocker, an angiotensin enzyme inhibitor, alpha receptor blocking drugs, beta receptor blocking drugs, antianginal antihypertensine drugs, digitalis drugs, hemorheologic drugs, inotropic drugs, myocardial infarction prophylaxis drugs, cerebral vasodilators, coronary vasodilators, peripheral vasodilators, and vasopressor drugs.

**Patentansprüche**

1. Verfahren zur Bereitstellung einer praktisch gleichmäßigen Medikamentenfreisetzungsrate aus einer Dosierungsform, worin die Dosierungsform eine Zusammensetzung umfaßt, welche eine Dosis eines Medikaments und ein hydrophiles Polymer umfaßt; worin das Verfahren (1) das Formulieren der Zusammensetzung mit einem Medikament, das eine Teilchengröße von weniger als 150 µm aufweist, und (2) das Formulieren der Zusammensetzung mit einem hydrophilen Polymer von weniger als 250 µm umfaßt; wobei durch gleichzeitige Anwesenheit von (1) und (2) in der Zusammensetzung das Medikament bei einer praktisch gleichmäßigen Freisetzungsrate aus der Dosierungsform abgegeben wird.

2. Verfahren nach Anspruch 1, worin die Zusammensetzung von einer Wand umschlossen ist, welche Vorrichtungen zur Freisetzung des Medikaments aus der Dosierungsform umfaßt.

3. Verfahren nach entweder Anspruch 1 oder 2, worin die Zusammensetzung von einer Außenwand und einer inneren Unterschicht umgeben ist mit Vorrichtungen in der Dosierungsform zur Freisetzung des Medikaments aus der Dosierungsform.

4. Verfahren zur Bereitstellung einer praktisch gleichmäßigen Medikamentenfreisetzungsrate aus einer Dosierungsform, worin die Dosierungsform umfaßt: eine Medikamentenschicht, die eine Dosis eines Medikaments und ein hydrophiles Polymer umfaßt, und eine Dispensierschicht, welche Vorrichtungen zur Abgabe der Medikamentenschicht aus der Dosierungsform umfaßt; worin das Verfahren das Formulieren der Medikamentenschicht mit einem Medikament, das eine Teilchengröße bis zu 150 µm besitzt und einem hydrophilen Polymer, das eine Teilchengröße bis zu 250 µm besitzt, umfaßt, wobei durch Zusammenwirken der Medikamententeilchen und der hydrophilen Polymerteilchen und der Dispensierschicht, die die Medikamentenschicht unterstützt, das Medikament bei einer praktisch gleichmäßigen Freisetzungsrate aus der Dosierungsform abgegeben wird.

5. Verfahren nach Anspruch 4, worin das hydrophile Polymerteilchen mit dem Medikamententeilchen als pharmazeutischer Träger zur Abgabe des Medikaments aus der Dosierungsform zusammenwirkt.

6. Verfahren nach entweder Anspruch 4 oder 5, worin die Dispensierschicht das Verdrängen der Medikamentenschicht aus der Dosierungsform unterstützt.

7. Verfahren nach einem der Ansprüche 4 bis 6, worin die Wand sowohl die Medikamentenschicht als auch die Dispensierschicht umschließt, und Vorrichtungen zur Freisetzung des Medikaments aus der Dosierungsform umfaßt.

8. Verfahren nach einem der Ansprüche 4 bis 7, worin eine Wand die Medikamentenschicht und die Dispensierschicht und eine Unterschicht zwischen der Wand und der Medikamentenschicht und der Dispensierschicht umgibt, und die Dosierungsform Ausgangsvorrichtungen zur Freisetzung des Medikaments aus der Dosierungsform umfaßt.

9. Dosierungsform zur Abgabe eines Medikaments, worin die Dosierungsform umfaßt:

 (a) eine Zusammensetzung;
 (b) eine Dosis eines Medikaments mit weniger als 150 um in der Zusammensetzung;
 (c) ein hydrophiles Polymer mit weniger als 250 µm in der Zusammensetzung;
 (d) eine Wand, welche eine Zusammensetzung umfaßt, die für den Durchfluß der Flüssigkeit, die die Dosis des Medikaments und das hydrophobe Polymer umgibt, durchlässig ist; und
 (e) Vorrichtungen in der Wand zur Abgabe des Medikaments bei einer praktisch gleichmäßigen Rate aus der Dosierungsform.

10. Dosierungsform zur Abgabe eines Medikaments, worin die Dosierungsform umfaßt:

(a) eine Medikamentenzusammensetzung;
(b) eine Dosis eines Medikaments mit weniger als 150 um in der Medikamentenzusammensetzung;
(c) ein hydrophiles Polymer mit weniger als 250 μm in der Medikamentenzusammensetzung;
(d) eine Schicht, die die Medikamentenzusammensetzung umgibt, und welche Vorrichtungen zur verzögerten Freisetzung des Medikaments aus der Medikamentenzusammensetzung umfaßt;
(e) eine Wand, welche eine Zusammensetzung umfaßt, die die Schicht umgibt; und
(f) Vorrichtungen in der Dosierungsform zur zeitabhängigen Abgabe des Medikaments aus der Dosierungsform.

11. Dosierungsform zur oralen Abgabe an einen Patienten, der das Medikament benötigt, worin die Dosierungsform umfaßt:

(a) eine Medikamentenzusammensetzung, die ein Medikament mit einer Teilchengröße bis zu und einschließlich 150 μm und einen hydrophilen Polymerträger mit einer Teilchengröße bis zu und einschließlich 250 μm für das Medikament umfaßt;
(b) eine Verdrängungszusammensetzung im Kontakt mit der Medikamentenzusammensetzung, die ein Polymer umfaßt, das in Gegenwart von Flüssigkeit zur Verdrängung der Medikamentenzusammensetzung aus der Dosierungsform expandiert;
(c) eine Wand, die das Medikament und die Verdrängungszusammensetzung umgibt und für den Durchfluß der Flüssigkeit durchlässig ist; und
(d) Vorrichtungen in der Dosierungsform zur zeitabhängigen Abgabe des Medikaments aus der Dosierungsform bei einer praktisch gleichmäßigen Rate.

12. Dosierungsform nach Anspruch 11, worin eine medikamentenfreie Schicht zwischen der Wand, dem Medikament und der Verdrängungszusammensetzung angeordnet ist, wodurch das Medikament und die Verdrängungszusammensetzung umschlossen sind, um den Durchfluß der Flüssigkeit in die Dosierungsform zu verringern.

13. Dosierungsform nach einem der Ansprüche 9 bis 12, worin das Medikament ausgewählt ist aus mindestens einem Mitglied der Gruppe, die aus Verapamil, Nifedipin, Nilvadipin, Flunarizin, Nimodipin, Diltiazem, Nicardipin, Nitredipin, Nisoldipin, Felodipin, Amlodipin, Isradipin, Cinnarizin, Fendilin, Ramipril, Fusinopril, Altiopril, Benazepril, Libenzapril, Alacepril, Cialzapril, Cilazaprilat, Perindopril, Zofenopril, Inalapril, Lisinopril, Imidapril, Spirapril, Rentiapril, Captopril, Delapril, Olindapril, Indalapril und Quinapril besteht.

14. Dosierungsform nach einem der Ansprüche 9 bis 12, worin die Medikamentenzusammensetzung ausgewählt ist aus mindestens einem Mitglied der Gruppe, die aus Prazosin, Clonidin, Pinacidil, Alfuzosin und Enalaprilat besteht.

15. Dosierungsform nach einem der Ansprüche 9 bis 12, worin das Medikament weiterhin ein Antioxidationsmittel umfaßt.

16. Dosierungsform nach einem der Ansprüche 9 bis 12, worin die Medikamentenzusammensetzung weiterhin ein oberflächenaktives Mittel umfaßt.

17. Dosierungsform nach Anspruch 11, worin das Medikament ausgewählt ist aus mindestens einem Mitglied der Gruppe, die aus einem Calciumkanalblocker, einem Angiotensin-Enzyminhibitor, Alpha-Rezeptorenblockern, Beta-Rezeptorenblockern, blutdrucksenkenden Medikamenten gegen Angina, Digitalismedikamenten, hämorheologischen Medikamenten, inotropen Medikamenten, prophylaktischen Medikamenten gegen Herzinfarkt, Gehirnvasodilatatoren, Kornarvasodilatatoren, peripheren Vasodilatatoren und vasopressorischen Medikamenten besteht.

**Revendications**

1. Procédé pour fournir une vitesse de libération de médicament sensiblement uniforme à partir d'une forme posologique, suivant lequel la forme posologique comprend une composition comprenant une dose de médicament et un polymère hydrophile ; dans lequel le procédé comprend les opérations consistant à (1) formuler la composition avec un médicament possédant une dimension inférieure à 150 microns, et (2) formuler la composition avec un polymère hydrophile de moins de 250 microns ; ce par quoi, par la coprésence de (1) et (2) dans la composition, le médicament est administré à une vitesse de libération sensiblement uniforme à partir de la forme posologique.

2. Procédé selon la revendication 1, dans lequel la composition est enveloppée par une paroi comprenant des moyens pour libérer le médicament à partir de la forme posologique.

**3.** Procédé selon l'une des revendications 1 et 2, dans lequel la composition est entourée par une paroi extérieure et une sous-couche intérieure, avec des moyens dans la forme posologique pour libérer le médicament à partir de la forme posologique.

**4.** Procédé pour fournir une vitesse de libération de médicament sensiblement uniforme à partir d'une forme posologique, suivant lequel la forme posologique comprend : une couche de médicament comprenant une dose de médicament et un polymère hydrophile et une couche d'administration comprenant des moyens pour administrer la couche de médicament à partir de la forme posologique ; dans lequel le procédé comprend les opérations consistant à formuler la couche de médicament avec un médicament possédant une dimension de particule allant jusqu'à 150 microns et un polymère hydrophile possédant une dimension de particule allant jusqu'à 250 microns ; ce par quoi, par la coopération des particules de médicament et des particules de polymère hydrophile et de la couche d'administration aidant la couche de médicament, le médicament est administré à une vitesse de libération sensiblement uniforme à partir de la forme posologique.

**5.** Procédé selon la revendication 4, dans lequel les particules de polymère hydrophile coopèrent avec les particules de médicament en tant que support pharmaceutique pour administrer le médicament à partir de la forme posologique.

**6.** Procédé selon l'une des revendications 4 et 5, dans lequel la couche d'administration aide à déplacer la couche de médicament à partir de la forme posologique.

**7.** Procédé selon l'une quelconque des revendications 4 à 6, dans lequel une paroi enferme à la fois la couche de médicament et la couche d'administration et comprend des moyens pour libérer le médicament à partir de la forme posologique.

**8.** Procédé selon l'une quelconque des revendications 4 à 7, dans lequel une paroi entoure la couche de médicament et la couche de distribution, et une une sous-couche entre la paroi et la couche de médicament et la couche de distribution, et la forme posologique comprend des moyens de sortie pour libérer le médicament à partir de la forme posologique.

**9.** Forme posologique pour l'administration d'un médicament, dans laquelle la forme posologique comprend :

   (a) une composition ;
   (b) une dose de médicament de moins de 150 microns dans la composition ;
   (c) un polymère hydrophile de moins de 250 microns dans la composition ;
   (d) une paroi comprenant une composition perméable au passage de fluide qui entoure la dose de médicament et le polymère hydrophobe ; et
   (e) des moyens dans la paroi pour administrer le médicament à une vitesse sensiblement uniforme à partir de la forme posologique.

**10.** Forme posologique pour l'administration d'un médicament, dans laquelle la forme posologique comprend :

   (a) une composition de médicament ;
   (b) une dose de médicament de moins de 150 microns dans la composition de médicament ;
   (c) un polymère hydrophile de moins de 250 microns dans la composition de médicament ;
   (d) un revêtement qui entoure la composition de médicament comprenant des moyens pour retarder la libération de médicament à partir de la composition de médicament ;
   (e) une paroi comprenant une composition qui entoure le revêtement ; et
   (f) des moyens dans la forme posologique pour administrer le médicament à partir de la forme posologique au cours du temps.

**11.** Forme posologique pour administrer un médicament par voie orale à un patient ayant besoin du médicament, dans lequel la forme posologique comprend :

   (a) une composition de médicament comprenant un médicament ayant une dimension de particule allant jusqu'à et y compris 150 microns, et un support polymère hydrophile ayant une dimension de particule ayant jusqu'à et y compris 250 microns pour le médicament ;
   (b) une composition de déplacement en contact avec la composition de médicament comprenant un polymère qui se dilate en présence de fluide pour déplacer la composition de médicament à partir de la forme posologique ;
   (c) une paroi qui entoure le médicament et la composition de déplacement et est perméable au passage du fluide ; et
   (d) des moyens dans la forme posologique pour administrer le médicament à partir de la forme posologique à une vitesse sensiblement uniforme au cours du temps.

**12.** Forme posologique selon la revendication 11, dans laquelle un revêtement exempt de médicament est disposé entre la paroi, et la composition de médicament et la composition de déplacement, entourant de cette façon la composition de médicament et la composition de déplacement de façon à ralentir le passage de fluide dans la forme posologique.

**13.** Forme posologique selon l'une quelconque des revendications 9 à 12, dans laquelle le médicament est choisi parmi au moins l'un des médicaments suivants : le vérapamil, la nifédipine, la nilvadipine, la flunarizine, la nimodipine, le diltiazem, la nicardipine, la nitrédipine, la nisoldipine, la félodipine, 1'amlodipine, l'isradipine, la cinnarizine, le fendiline, le ramipril, le fusinopril, l'altiopril, le bénazépril, le libenzapril, l'alacépril, le cialzapril, le cilazaprilat, le perindropil, le zofenopril, l'inalapril, le lisinopril, l'imidapril, le spirapril, le rentiapril, le captopril, le délapril, l'olindrapril, l'indalapril et le quinapril.

**14.** Forme posologique selon l'une quelconque des revendications 9 à 12, dans laquelle le médicament est choisi parmi au moins l'un des médicaments suivants : la prazosine, la clonidine, le pinacidil, l'alfuzosine et l'énalaprilat.

**15.** Forme posologique selon l'une quelconque des revendications 9 à 12, dans laquelle la composition de médicament comprend en outre un antioxydant.

**16.** Forme posologique selon l'une quelconque des revendications 9 à 12, dans laquelle la composition de médicament comprend en outre un agent tensioactif.

**17.** Forme posologique selon la revendication 11, dans laquelle le médicament est choisi parmi au moins l'un des médicaments suivants : un bloqueur des canaux calciques, un inhibiteur de l'enzyme de conversion de l'angiotensine, les médicaments bloqueurs des alpharécepteurs, les médicaments bloqueurs des bêtarécepteurs, les médicaments antihypertenseurs antiangineux, les médicaments digitaliques, les médicaments hémorhéologiques, les médicaments inotropes, les médicaments de prophylaxie de l'infarctus du myocarde, les vasodilatateurs cérébraux, les vasodilatateurs coronariens, les vasodilatateurs périphériques et les médicaments vasopresseurs.

# F I G. I

EP 0 907 358 B1

FIG.2

Release rate

% dev. from mean

% dev. from total mean release rate

28.43 27.58   21.41   26.94   28.44   24.96   28.03   26.8   26.78   30.66

Release rate

FIG.3

EP 0 907 358 B1